# EUROPEAN PATENT SPECIFICATION

(11) **EP 4 013 369 B1**
(45) Date of publication and mention of the grant of the patent: **15.01.2025**
(21) Application number: 20751600.6
(22) Date of filing: 12.08.2020
(51) Int. Cl.: A61H 35/04, A61M 3/02, A61M 15/08

(54) **NOZZLE FOR A CONTAINER FOR INTRANASAL ADMINISTRATION**
DÜSE FÜR EINEN BEHÄLTER ZUR INTRANASALEN VERABREICHUNG
BUSE DE RÉCIPIENT POUR ADMINISTRATION INTRANASALE

(30) Priority: 14.08.2019 EP 19191644
(43) Date of publication of application: 22.06.2022
(73) Proprietor: Haleon CH SARL, 1197 Prangins (CH)
(72) Inventor: BANERJEE, Sriman, 1197 Prangins Nyon (CH); BHUNIA, Manas Kumar, 1197 Prangins Nyon (CH)
(74) Representative: Haleon Patent Department
(86) International application number: PCT/EP2020/072664
(87) International publication number: WO 2021/028495

(56) References cited:
- EP-A1- 2 210 580
- WO-A1-2011/075581
- WO-A2-2008/122791
- GB-A- 2 364 321
- US-A1- 2005 211 241
- US-A1- 2011 301 572
- US-A1- 2016 331 916
- US-A1- 2018 126 063

## Description

This invention relates to a nozzle for a container for the intranasal administration of a liquid formulation, in particular for the intranasal administration of a nasal spray or nasal wash, and to a packaging comprising such nozzle.

Intranasal administration of a liquid formulation, for example a drug formulation or a medicinal product formulation frequently employs a primary packaging for such formulation that comprises a container and a nozzle that is suitable to be introduced to a user's nostril and from which such liquid formulation is dispensed into the nostril.

Numerous packaging solutions comprising nozzles suitable for intranasal administration of a liquid formulation are known. For example, EP2335759 (B1) discloses an apparatus and method for preparing a pH balanced saline solution and using the saline solution for rinsing a nasal passage wherein the apparatus comprises a container having flexible sidewalls and an opening for a removable cap, that cap being pressed against a nostril and having an opening from which the saline solution is dispensed into a user's nasal passage.

CN203885832U discloses a nasal irrigation device that comprises a flexible container bottle, a flexible nozzle, a bottle cap and a flow guide tube wherein the flexible nozzle is connected with the top of the bottle cap and wherein the nasal irrigation fluid is ejected out of the flexible nozzle.

GB2364321A discloses use of a thermoplastic elastomer co-moulding for forming an outlet tip of a nasal actuator, wherein a rigid core of polymeric plastics or metal defines an outlet orifice and is overlaid by a layer of the thermoplastic elastomer.

US2011301572A1 discloses an ear medication delivery device for delivering a treatment liquid and retaining the treatment liquid within an ear canal of a patient. An earplug in an embodiment thereof is made in one piece of somewhat pliable hypoallergenic material that is non-reactive with the tissue of the external ear canal like compressible resilient elastomeric foam, rubber, silicone, silicone putties, vinyl or acrylics by techniques such as molding and machining.

When adjusting nozzles to suit the intranasal administration of a liquid formulation, there are several difficulties that one is likely to encounter.

Packaging materials must suffice various requirements before they can be used in the field of health care, pharmaceuticals or cosmetics. A Packaging for liquid formulations is considered particularly critical, and therefore regulatory authorities have established quality criteria for packaging materials that must be met to guarantee patient and consumer safety. Examples of such criteria are migration studies and interaction studies and limits for leachable and extractable compounds. The qualification of any packaging material that is in contact with a liquid formulation is costly and time-consuming and thus limiting the range of possible materials for the innovator in the field.

Dimension and shape of the nozzle must be adjusted to reflect the anatomy of the nostril and the nasal cavity and the intranasal region that is targeted. For example, a nozzle that is aimed to topically and locally administer a liquid formulation onto the mucosa of the sinuses will have a different shape than a nozzle that is designed to administer a liquid formulation into the systemic pathway via the respiratory mucosa.

The anatomy of the nostril and the nasal cavity also present challenges. The skin around the nostril as well as the intranasal mucosa are very sensitive. Especially when a patient suffers conditions including nasal symptoms such as runny nose, the skin and mucosa in the nasal region can be painfully irritated and inflamed. Therefore, it is a desirable feature of a nozzle for the intranasal administration of a liquid formulation to provide a soft surface feel that is gentle to the nostril skin, the intranasal skin and the intranasal mucosa, and that is not causing further irritation of the skin in the nasal region.

The small size of the nasal vestibule makes it difficult to introduce a nozzle. This especially applies for users with impaired motor skills or when one person aims to administer a liquid formulation to another person. Flexible nozzles made from a rubber or an elastic plastic material may be more suitable for such purposes, as they may prick and hurt less than rigid nozzles when the administering person tangents the surroundings of the nostril, for example the ala of the nose, the nasal septum or columella, the philtrum or the outer parts of the nostril. However, when the administering person does not introduce the nozzle directly into the nostril with the nozzle being well centered, such flexible nozzles may bend over instead of gliding into the nostril. Multiple attempts may be necessary for a successful introduction into the nostril. Additionally, rubber and elastic plastics need to fulfill regulatory requirements for plastic immediate packaging materials, which results in the disadvantages set out above.

Therefore, the problem underlying the present invention is to provide a nozzle and a for the intranasal administration of a liquid formulation which has enough stability to glide into the nostril instead of bending over, even if the administering person tangents the surroundings of the nostril thus making the introduction into the nostril easier, but still provides a soft surface feel and a gentle contact to the anatomy of the nose, the nostril and the surroundings of the nostril, while still being cost-effective.

This problem is solved by the subject matter of the invention as defined in the appended claims.

According to the invention there is provided a nozzle for a container for intranasal administration of a liquid as defined in the independent claim 1.

In one embodiment, the first material is a thermoplastic material.

In one embodiment, the first material is a polyolefin.

In one embodiment, the first material is a low-density polyethylene.

In one embodiment, the first material has a Shore A hardness of 80 - 100.

In one embodiment, the outer surface of the nozzle body and the nozzle cover have an approximately semi-spherical shape.

In one embodiment, the nozzle cover has an orifice which flushes with the upper end of the nozzle body.

In one embodiment, the nozzle cover has a flat rim surrounding an orifice of the nozzle cover and the flat rim flushes with the upper end of the nozzle body.

In one embodiment, an orifice of the nozzle cover is aligned with the orifice of the nozzle body, and the orifice of the nozzle cover has a larger diameter than the orifice of the nozzle body.

In one embodiment, the nozzle is designed such that the liquid formulation is not in contact with the second material when dispensed from the nozzle.

According to the invention, there is provided a packaging for a liquid formulation for intranasal administration comprising a container and such nozzle.

In one embodiment, the container is a squeeze bottle.

There is also described a product comprising such a packaging and a liquid formulation in said packaging.

In one embodiment, the liquid formulation is a nasal wash formulation. In one embodiment, the liquid formulation is a nasal spray formulation.

The nozzle and the packaging of the present invention are suitable for the intranasal administration of liquid formulations, for example drug product formulations or medicinal product formulations. These include for example medicated or non-medicated nasal sprays, rinses or washes. The teachings of the present invention can be applied to all nozzles for the intranasal administration of liquid formulations and therefore to all packagings comprising such nozzles and to all products comprising such packagings. The nozzle and the packaging of the present disclosure are especially beneficial for nasal wash formulations.

The channel traversing the nozzle body allows a liquid formulation to flow through it and to be dispensed through the orifice in the upper end of the nozzle body.

The rigid plastic material of the nozzle body ensures that the nozzle has enough stability to be introduced into or to glide into the nostril of the user without bending over, even if the administering person tangents the surroundings of the nostril, thus facilitating the introduction into the nostril. It also allows the nozzle body to be easily mounted on or connected to other parts, for example to a container or to a connecting piece connecting the nozzle and an actual container. The material of the nozzle body is in contact with the liquid formulation and therefore needs to fulfill regulatory requirements for plastic immediate packaging materials for liquid dosage forms. Rigid plastic materials meet these requirements more often than rubber or elastic plastic materials, namely because they do in general not contain plasticizers that could migrate into the liquid formulation. This is one of the reasons why rigid plastic materials are preferred packaging materials in the field of health care, pharmaceuticals or cosmetics, and the manufacturer may choose from a broad spectrum of suitable rigid plastic materials commercially available.

The elastic plastic material of the nozzle cover provides a soft surface feel that is gentle to the nostril skin, the intranasal skin and the intranasal mucosa and is not causing further irritation of the skin in the nasal region. Additionally, the elastic plastic material reduces the risk of pricking, hurting and injury. This ensures an easier and safer introduction into the nostril, especially for users with impaired motor skills or when a person such as a caretaker or a parent aims to administer a liquid formulation to another person. In comparison with nozzles having a rigid plastic material surface, the elastic plastic material of the nozzle cover also provides a better sealing of the nostril and ensures that the liquid formulation does not immediately flow back out of the nose.

Using a first, rigid plastic material for the nozzle body and a second, elastic plastic material for the nozzle cover combines the advantages of both materials as set out above.

According to the present invention, the elastic plastic material of the nozzle cover is not in contact with the orifice.

This eliminates the risk of an interaction between the liquid formulation and the elastic plastic material, such as migration of plasticizers or other ingredients of the elastic plastic material into the liquid formulation. Therefore, a qualification of the elastic plastic material as plastic immediate packaging material for medicinal products is not necessary, making the qualification of the total packaging less costly and less time consuming and making it possible to choose from a broader range of materials for the elastic plastic material.

In a preferred embodiment, the nozzle cover has an orifice which flushes with the upper end of the nozzle body. The nozzle cover only covers an area of the outer surface of the nozzle body and not an upper area or inner area of the nozzle body. The nozzle cover does not project above the upper end of the nozzle body.

In another preferred embodiment, the nozzle cover has a flat rim surrounding an orifice of the nozzle cover and the flat rim flushes with the upper end of the nozzle body. The rim is the uppermost part of the nozzle cover and does not project beyond or over the upper end of the nozzle body. The nozzle cover ending in the flat rim, rather than for example pointy, aids prevention of stripping of the two layers.

According to the invention, a flow channel for the liquid formulation is formed in the nozzle. The flow channel is lined by the first material only. When the nozzle is used to expel a liquid formulation from a container, the liquid formulation flows from the container, optionally through a dip tube, through the channel traversing the nozzle body and out of the orifice of the nozzle body, without any contact to the nozzle cover.

In a preferred embodiment, and as shown in the figures, an orifice of the nozzle cover is aligned with the orifice of the nozzle body and has a larger diameter than the orifice of the nozzle body. Thereby, a distance between the two orifices is created which separates the nozzle cover from the orifice of the nozzle body. The distance is filled by the nozzle body, i.e. the first material. Thereby, the liquid formulation is not in contact with the second material, even after expulsion from the orifice of the nozzle body.

Preferably, the orifice of the nozzle cover is arranged concentric with the orifice of the nozzle body. Concentric arrangement of the two orifices provides that the difference in diameters, i.e. the distance between, the two orifices is consistent throughout the circumferences of the orifices.

Preferably, the orifice of the nozzle cover and the orifice of the nozzle body are on the same height of a lengths of the nozzle in direction of a flow channel of the liquid formulation. Preferably, the orifice of the nozzle body and the orifice of the nozzle cover are aligned with the upper end of the nozzle body. This arrangement avoids edges, protrusions or depressions where material or dirt could accumulate.

In a preferred embodiment, the nozzle is designed such that the liquid formulation is not in contact with the second material when dispensed from the nozzle. A flow channel of the liquid formulation as well as a flow direction of the liquid formulation after expulsion are free from the second material of the nozzle cover. The nozzle cover is recessed from the orifice of the nozzle body such that a liquid formulation, when expelled from the orifice of the nozzle body, scatters without contact to the nozzle cover.

These preferred constructions all increase the security that the liquid formulation that is dispensed from the orifice of the nozzle body is not in contact with the nozzle cover, but only with the nozzle body, i.e. the first material.

According to the invention, the nozzle is produced by bi-injection moulding. This method allows to produce in a single production step both the nozzle body and the nozzle cover and to provide a permanent bond between the rigid plastic material of the nozzle body and the elastic plastic material of the nozzle cover. Bi-injection moulding is a well-established method for the processing of plastic materials and therefore a wealth of materials suitable for this injection-moulding, and in particular for bi-injection moulding, has been developed and is commercially available.

In one preferred embodiment, the first material is a thermoplastic material. Thermoplastic materials are preferred because they can be processed by injection moulding and bi-injection moulding, are recyclable, sufficiently rigid, stable and lightweight. Their characteristics such as colour, hardness and flexibility can easily be adjusted by mixture of different plastics and/ or by the addition of additives.

In a more preferred embodiment, the first material is a polyolefin, most preferred a low-density polyethylene. Polyolefins show desirable chemical and mechanical stability and can conveniently be processed by standard methods in the art, for example injection moulding and bi-injection moulding. Low-density polyethylene is preferred because containers made from this material show excellent containment of liquids and impermeability, thus providing the desired protection for the contained product. It is a well-established plastic material in the fields of food, cosmetics and pharmaceutics and a wealth of low-density polyethylene raw materials are commercially available, including pharmaceutical grade quality.

According to the technology, the second material may be an elastomer, a thermoplastic elastomer or a thermoplastic rubber. Typically, elastomers and thermoplastic elastomers are elastic and flexible materials that show the desired characteristic of the soft surface feel as described above. Elastomer and thermoplastic elastomer raw materials that may be processed by injection moulding and bi-injection moulding are available.

According to the invention, the second material is a thermoplastic elastomer, most preferred injection-moulding grade of thermoplastic elastomer. Thermoplastic elastomers are a class of copolymers or a physical mix of polymers, such as a plastic and a rubber, that have thermoplastic as well as elastomeric properties. They can be processed with the same methods as thermoplastic materials, for example in the same production step, for example by bi-injection moulding. Thermoplastic materials and thermoplastic elastomers, when injected into a mould subsequently, may mix to a certain degree on the interface between the two materials, forming a permanent bond between the two materials upon cooling. Additionally, moulding of thermoplastic elastomers does not require an additional step of cross-linking that would be required for elastomers, what makes thermoplastic elastomers recyclable.

Softness and hardness of different plastic materials may suitably be assessed with a Shore durometer and applying the American Society for Testing and Materials' standard ASTM D2240 ("Standard Test Method for Rubber Property - Durometer Hardness"). Results are expressed in values on the Shore hardness scale. An indentor is forced into a material under specified conditions. The indentation hardness negatively correlates to the penetration and is dependent on different material properties. Low values indicate softer materials and less resistance. For soft materials, suitably the Shore durometer A with a truncated indentor is used to determine the hardness on the Shore scale A, which herein is referred to as Shore A hardness.

In one preferred embodiment, the nozzle body may have a Shore A hardness of 80 - 100. Nozzle bodies of this hardness exhibit the stability that is required for the introduction into the nostril and the connecting to a container as set out above, exhibit desired containment and still are elastic enough to not break.

According to the invention, the nozzle cover has a Shore A hardness of 20 - 40, more preferred a Shore A hardness of 25-35, even more preferred a Shore A hardness of 28-32. Nozzle covers of this hardness exhibit the soft surface feel and the benefits as set out above and still are sufficiently resistant to mechanical forces. Additionally, they exhibit advantageous conjunction when processed with a thermoplastic material by bi-injection moulding.

In one preferred embodiment the outer surface of the nozzle body and the nozzle cover have an approximately semi-spherical shape. A semi-spherical shape avoids sharp edges that could provoke lesions of the nostril skin, the intranasal skin and the intranasal mucosa. Additionally, such a shape is easy to introduce into the nostril because the nostril also has an approximately round cross-section. Typically, the cross-section of the nozzle may be circular, and the nozzle may be rotationally symmetric around a central axis. The dimension of the nozzle, i.e. its diameter, length, and tapering, may suitably be designed in such way to partially fit into the nostril and to seal the nostril when introduced, thereby avoiding that the nasal wash immediately flows back out of the nose.

The nozzle, when introduced into a nostril, thus seals the nostril. This is especially beneficial for nozzles for nasal wash formulations, because a backflow of the wash through the same nostril is thereby prevented. Furthermore, sealing of the nostril facilitates building up of the pressure that is needed to direct the nasal wash formulation through the nasal cavities and out through the opposite nostril.

In another aspect of the invention there is disclosed a packaging for the intranasal administration of a liquid formulation comprising a container and a nozzle as described above. Said packaging may include a removable closure cap.

In one preferred embodiment, the container is a squeeze bottle. The nozzle can be mounted directly on the squeeze bottle, without a need for any further dosing or dispensing mechanism. Alternatively, the container can be a conventional bottle, and the nozzle can then be mounted on a dispensing or dosing mechanism, or the dispensing or dosing mechanism can be accommodated inside the nozzle. The nozzle is suitable for use in combination with metered-dose spray pumps, continuous spray pumps or any other pump, dosing mechanism and dispensing mechanism that is suitable for intranasal administration of liquid formulations.

Typically, the design and the dimensions of the container may be adjusted to address the requirements that come along with different liquid formulations for intranasal administration. Typically, the container can contain a volume of 10 ml to 150 ml. For example, the container may contain a volume of around 100 ml of a liquid formulation of a nasal wash and a volume of around 10ml for a nasal spray.

Further, there is disclosed a product comprising a packaging as described above.

For nasal wash products, a relatively large dose of liquid formulation has to be introduced into the nostril and exact dosing is not needed. Therefore, said products are often sold in squeeze bottles. For nasal spray products, for example non-medicated nasal spray products, a smaller dose and controlled droplet size of the spray are required. Therefore, these products typically are contained in solid bottles and dispensed with a spraying mechanism, for example a continuous spray pump. For medicinal products and medicated nasal sprays, exact dosing and a controlled droplet size are required. Therefore, these products are typically contained in solid bottles and dispensed with a spray mechanism and a dosing mechanism, for example a metered-dose spray pump.

A preferred embodiment of the invention will now be described by way of example only with reference to the accompanying drawings.

### Brief description of the drawings.

Fig. 1 shows a perspective view of a packaging according to the invention comprising a bi-injection nozzle of the present invention and a squeeze bottle.
Fig. 2 shows an enlarged perspective individual view of the bi-injection nozzle of Fig. 1.
Fig. 3 shows a cross-section of the bi-injection nozzle of Fig. 2.
Fig. 4 shows a perspective view of the bi-injection nozzle of Fig. 2 with the nozzle cover omitted.
Fig. 5 shows a perspective individual view of the nozzle cover of the bi-injection nozzle of Fig. 2.

### Detailed description of a preferred embodiment of the invention.

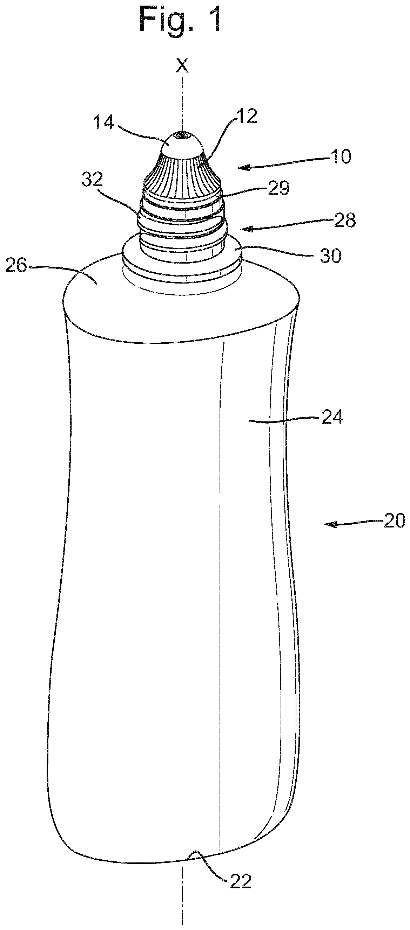

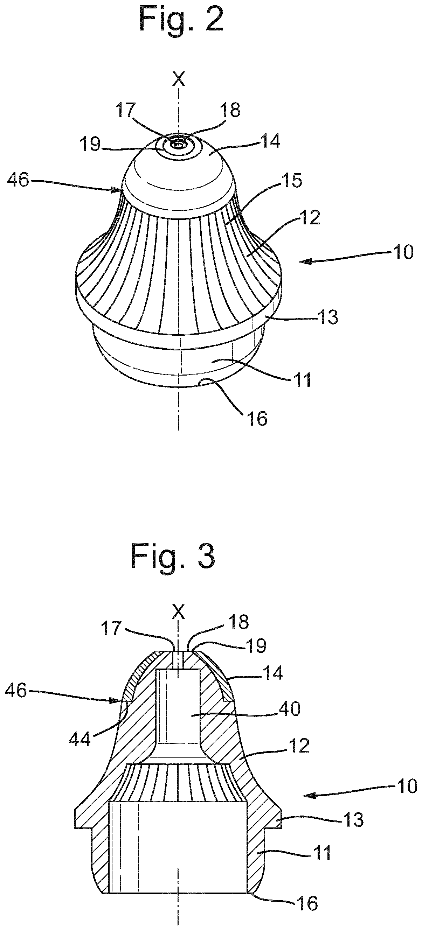

Referring to Fig. 1, a packaging for liquid formulations for intranasal administration comprises a squeeze bottle 20, made from a low-density polyethylene and typically housing a volume of 10-150 ml of a liquid formulation. The squeeze bottle 20 has an oval cross-section with a flat bottom 22 on which the bottle can stand, and, when looking at it from the bottom to the top, tapers towards a waist 24 from which it expands again to a shoulder 26. This shape of the squeeze bottle 20 is an ergonomic design that improves grip and reduces the pressure that has to be applied to the squeeze bottle 20 to expel a liquid formulation. From the shoulder 26 arises a bottle neck 28 that comprises a cap shoulder 30 and a thread 32. A cap (not shown in the figures) can be screwed onto the bottle neck 28, with the cap shoulder 30 forming a stopper for such a cap. A nozzle 10 is mounted onto the bottle neck 28 by means of a press fit connection. The nozzle 10 consists of two components which have been moulded together in a bi-injection moulding process, namely a nozzle body 12 and a nozzle cover 14, and will be described more in detail below referring to Fig. 2.

Fig. 2 shows an enlarged perspective individual view of the bi-injection nozzle 10 of Fig. 1. The nozzle 10 is rotationally symmetric around an axis of symmetry X. The nozzle body 12 ends at its lower end 16 in a cylindrical press fit plug 11 that provides for a tight sealing between the nozzle body 12 and the bottle neck 28 of the squeeze bottle 20 when fitted into the equally cylindrical bottle neck 28. From the press fit plug 11 protrudes a base 13 of the nozzle body 12 which will sit on a rim 29 of the bottle neck 28 when the nozzle is mounted on the squeeze bottle 20. The base 13 is the widest part of the nozzle body 12 which is tapered towards an upper end 18 and ends in an approximately semi-spherical shape that is suitable to be introduced into a user's nostril. The tapered shape is suitable to hinder the nozzle 10 from being introduced too far into the user's nostril, and to form a tight seal between the nozzle 10 and the nostril when introduced. A channel 40, visible in the cross-section of Fig. 3 and further described below, traverses the nozzle body 12 and ends in an orifice 17 in the upper end 18. Through the channel 40 the liquid formulation can be expelled out of the orifice 17 of the nozzle body 12. A portion of the outer surface of the nozzle body 12 comprises facets 15 for an increased user appeal and a more pleasant surface touch. A top portion of the outer surface of the nozzle body 12 is covered by a nozzle cover 14. The nozzle cover 14 reproduces the shape of the nozzle body 12, i.e. it has an approximately semi-spherical shape, and is dimensioned such that it can be entirely introduced into a user's nostril when introducing the nozzle 10. It provides a soft surface feel to the nostril skin, the intranasal skin and the intranasal mucosa. The nozzle cover 14 has an orifice 19 that is aligned with the orifice 17 of the nozzle body 12 and has a larger diameter than the orifice 17 of the nozzle body 12. The orifice 19 of the nozzle cover 14 having a larger diameter than the orifice 17 of the nozzle body 12 ensures that the nozzle cover 14 is not in contact with the orifice 17 of the nozzle body 12. It therefore assures that the liquid formulation that is dispensed from orifice 17 of the nozzle body 12 will not be in contact with the nozzle cover 14 but only with the nozzle body 12. The nozzle body 12 is made from an injection-moulding grade of low-density polyethylene. The nozzle cover 14 is made from an injection-moulding grade of thermoplastic elastomer, for example Medalist MD-12130, available from Teknor Apex Company, Mijnweg 1, 6167, AC Geleen, Netherlands, www.teknorapex.com. This thermoplastic elastomer has a Shore A hardness of 32 after one second and a Shore A hardness of 30 after 5 seconds when measured on a Shore A durometer applying the ASTM D2240 standard.

These plastic materials are preferred as they offer suitable Shore A hardness and allow efficient manufacture by bi-injection moulding.

Fig. 3 shows a cross-section of the bi-injection nozzle 10 of Fig. 2. It shows a channel 40 extending vertically through the axis of symmetry X of the nozzle body 12 and ending in the orifice 17 of the nozzle body 12 in the upper end 18 of the nozzle body 12. The orifice 19 of the nozzle cover 14 is aligned with the orifice 17 of the nozzle body 12 and has a larger diameter than the orifice 17 of the nozzle body 12, so that the nozzle cover 14 is not in contact with the orifice 17 of the nozzle body 12. The nozzle cover 14 covers an outer surface of the nozzle body 12 and extends from the upper end 18 of the nozzle body 12 to a circular rim 44 which is foreseen on the nozzle body 12. When moulded together in a bi-injection moulding process, the second material of the nozzle cover 14 fills the space above the circular rim 44 of the nozzle body 12 so as to form a circular borderline 46 of the first material of the nozzle body 12 and the second material of the nozzle cover 14. The rim 44 ensures an interface between the first and the second material big enough to provide adhesion and to prevent stripping of the two materials.

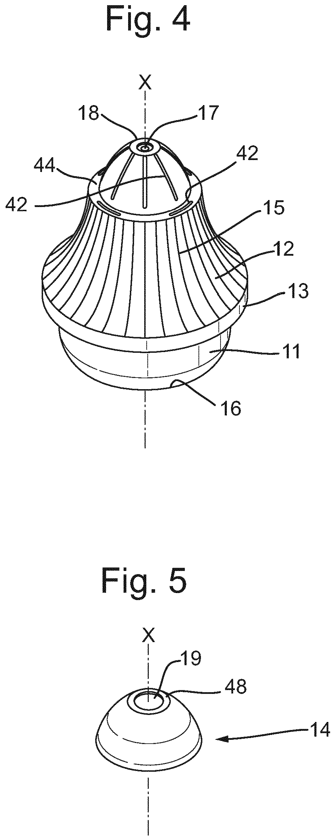

Fig. 4 shows a perspective view of bi-injection nozzle 10 of Fig. 2 with the nozzle cover 14 omitted. The upper end 18 of the nozzle body 12 comprises ribs 42 that protrude from the outer surface and the circular rim 44 of the nozzle body 12 that improve the adhesion between the nozzle body 12 and the nozzle cover 14 additionally to the inherent bonding obtained by bi-injection moulding.

Fig. 5 shows a perspective individual view of the nozzle cover 14 of the bi-injection nozzle 10 of Fig. 2. It shows that the orifice 19 of the nozzle cover 14 has a larger diameter than the orifice 17 of the nozzle body 12 as shown in Fig. 4. The nozzle cover 14 reproduces the shape of the nozzle body 12, i.e. it is rotationally symmetric around an axis of symmetry X and tapers in an approximately semi-spherical shape. A flat rim 48 borders the orifice 19 of the nozzle cover 14. This flat rim 48 provides an advantageous broad surface of adhesion for the second material of the nozzle cover 14 on the upper end 18 of the nozzle body 12 and prevents stripping of the two layers.

## Claims

1. A nozzle (10) for a container for intranasal administration of a liquid formulation, comprising a nozzle body (12) with an upper (18) end and a lower end (16), a channel (40) traversing the nozzle body (12) and ending with an orifice (17) in the upper end (18), and a nozzle cover (14) covering an area of the outer surface of the nozzle body (12) which is designed to be introduced into a user's nostril, wherein the nozzle body (12) is made out of a first material, and the nozzle cover (14) is made out of a second material, said first material being a rigid plastic material, and said second material being an elastic plastic material, and wherein the nozzle cover (14) is not in contact with the orifice (17), wherein the nozzle (10), when introduced into a nostril, seals the nostril, and wherein a flow channel for the liquid formulation is formed in the nozzle (10), and wherein the flow channel is lined by the first material only, **characterised in that** the nozzle (10) is produced by bi-injection moulding, and that the second material is a thermoplastic elastomer with a Shore A hardness of 20 - 40.

2. A nozzle (10) according to claim 1 wherein the first material is a thermoplastic material.

3. A nozzle (10) according to any of the preceding claims wherein the first material is a polyolefin.

4. A nozzle (10) according to any one of the preceding claims wherein the first material is a low-density polyethylene.

5. A nozzle (10) according to any of the preceding claims wherein the first material has a Shore A hardness of 80 - 100.

6. A nozzle (10) according to any of the preceding claims wherein the outer surface of the nozzle body (12) and the nozzle cover (14) have an approximately semi-spherical shape.

7. A nozzle (10) according to any of the preceding claims wherein the nozzle cover (14) has an orifice (19) which flushes with the upper end (18) of the nozzle body (12).

8. A nozzle (10) according to any of the preceding claims wherein the nozzle cover (14) has a flat rim (48) surrounding an orifice of the nozzle cover (19) and the flat rim (48) flushes with the upper end (18) of the nozzle body (12).

9. A nozzle (10) according to any of the preceding claims wherein an orifice of the nozzle cover (19) is aligned with the orifice of the nozzle body (17), and wherein the orifice of the nozzle cover (19) has a larger diameter than the orifice of the nozzle body (17).

10. A nozzle (10) according to any of the preceding claims wherein the nozzle is designed such that the liquid formulation is not in contact with the second material when dispensed from the nozzle (10).

11. A packaging for a liquid formulation for intranasal administration comprising a container and a nozzle (10) according to any of claims 1 to 10.

12. A packaging according to claim 11 wherein the container is a squeeze bottle (20).

## Patentansprüche

1. Düse (10) für einen Behälter zur intranasalen Verabreichung einer flüssigen Formulierung, aufweisend
einen Düsenkörper (12) mit einem oberen (18) Ende und einem unteren Ende (16),
einen Kanal (40), der den Düsenkörper (12) durchquert und mit einer Öffnung (17) im oberen Ende (18) endet, und
einer Düsenabdeckung (14), die einen Bereich der Außenoberfläche des Düsenkörpers (12) abdeckt, der dazu gestaltet ist, in das Nasenloch eines Benutzers eingeführt zu werden, wobei
der Düsenkörper (12) aus einem ersten Material hergestellt ist und die Düsenabdeckung (14) aus einem zweiten Material hergestellt ist, wobei
das erste Material ein starres Kunststoffmaterial ist und das zweite Material ein elastisches Kunststoffmaterial ist, und wobei
die Düsenabdeckung (14) nicht in Kontakt mit der Öffnung (17) ist, wobei
die Düse (10), wenn sie in ein Nasenloch eingeführt wird, das Nasenloch abdichtet, und wobei
ein Strömungskanal für die flüssige Formulierung in der Düse (10) ausgebildet ist, und wobei
der Strömungskanal nur durch das erste Material ausgekleidet ist, **dadurch gekennzeichnet,**
**dass** die Düse (10) durch Bi-Spritzgießen hergestellt ist, und
**dass** das zweite Material ein thermoplastisches Elastomer mit einer Shore A-Härte von 20 - 40 ist.

2. Düse (10) nach Anspruch 1, wobei das erste Material ein thermoplastisches Material ist.

3. Düse (10) nach einem der vorherigen Ansprüche, wobei das erste Material ein Polyolefin ist.

4. Düse (10) nach einem der vorherigen Ansprüche, wobei das erste Material ein Polyethylen niedriger Dichte ist.

5. Düse (10) nach einem der vorherigen Ansprüche, wobei das erste Material eine Shore-A-Härte von 80 - 100 aufweist.

6. Düse (10) nach einem der vorherigen Ansprüche, wobei die Außenoberfläche des Düsenkörpers (12) und die Düsenabdeckung (14) eine annähernd halbkugelförmige Form aufweisen.

7. Düse (10) nach einem der vorherigen Ansprüche, wobei die Düsenabdeckung (14) eine Öffnung (19) aufweist, die mit dem oberen Ende (18) des Düsenkörpers (12) fluchtet.

8. Düse (10) nach einem der vorherigen Ansprüche, wobei die Düsenabdeckung (14) einen flachen Rand (48) aufweist, der eine Öffnung der Düsenabdeckung (19) umgibt, und der flache Rand (48) mit dem oberen Ende (18) des Düsenkörpers (12) fluchtet.

9. Düse (10) nach einem der vorherigen Ansprüche, wobei eine Öffnung der Düsenabdeckung (19) mit der Öffnung des Düsenkörpers (17) ausgerichtet ist und wobei die Öffnung der Düsenabdeckung (19) einen größeren Durchmesser als die Öffnung des Düsenkörpers (17) aufweist.

10. Düse (10) nach einem der vorherigen Ansprüche, wobei die Düse so gestaltet ist, dass die flüssige Formulierung nicht in Kontakt mit dem zweiten Material ist, wenn sie aus der Düse (10) abgegeben wird.

11. Verpackung für eine flüssige Formulierung zur intranasalen Verabreichung aufweisend einen Behälter und eine Düse (10) nach einem der Ansprüche 1 bis 10.

12. Verpackung nach Anspruch 11, wobei der Behälter eine Quetschflasche (20) ist.

## Revendications

1. Buse (10) pour récipient pour l'administration intranasale d'une formulation liquide, comprenant un corps de buse (12) avec une extrémité supérieure (18) et une extrémité inférieure (16), un canal (40) traversant le corps de buse (12) et se terminant par un orifice (17) dans l'extrémité supérieure (18), et un couvercle de buse (14) recouvrant une zone de la surface extérieure du corps de buse (12) qui est conçue pour être introduite dans la narine d'un utilisateur, où le corps de buse (12) est constitué d'un premier matériau, et le couvercle de buse (14) est constitué d'un second matériau, ledit premier matériau étant un matériau plastique rigide, et ledit second matériau étant un matériau plastique élastique, et où le couvercle de buse (14) n'est pas en contact avec l'orifice (17), où la buse (10), lorsqu'elle est introduite dans une narine, ferme hermétiquement la narine, et où un canal d'écoulement pour la formulation liquide est formé dans la buse (10), et où le canal d'écoulement est revêtu du premier matériau seulement, **caractérisée en ce que** la buse (10) est produite par moulage par bi-injection, et le second matériau est un élastomère thermoplastique avec une dureté Shore A de 20 - 40.

2. Buse (10) selon la revendication 1, où le premier matériau est un matériau thermoplastique.

3. Buse (10) selon l'une quelconque des revendications précédentes, où le premier matériau est une polyoléfine.

4. Buse (10) selon l'une quelconque des revendications précédentes, où le premier matériau est un polyéthylène basse densité.

5. Buse (10) selon l'une quelconque des revendications précédentes, où le premier matériau a une dureté Shore A de 80 - 100.

6. Buse (10) selon l'une quelconque des revendications précédentes, où la surface extérieure du corps de buse (12) et le couvercle de buse (14) ont une forme approximativement semi-sphérique.

7. Buse (10) selon l'une quelconque des revendications précédentes, où le couvercle de buse (14) a un orifice (19) qui est au même niveau que l'extrémité supérieure (18) du corps de buse (12).

8. Buse (10) selon l'une quelconque des revendications précédentes, où le couvercle de buse (14) a un bord plat (48) entourant un orifice du couvercle de buse (19) et le bord plat (48) est au même niveau que l'extrémité supérieure (18) du corps de buse (12).

9. Buse (10) selon l'une quelconque des revendications précédentes, où un orifice du couvercle de buse (19) est aligné avec l'orifice du corps de buse (17), et où l'orifice du couvercle de buse (19) a un diamètre plus grand que l'orifice du corps de buse (17).

10. Buse (10) selon l'une quelconque des revendications précédentes, où la buse est conçue de telle sorte que la formulation liquide n'est pas en contact avec le second matériau lorsqu'elle est distribuée depuis la buse (10).

11. Emballage pour formulation liquide pour l'administration intranasale comprenant un récipient et une buse (10) selon l'une quelconque des revendications 1 à 10.

12. Emballage selon la revendication 11 où le récipient est un flacon souple (20).
